# EUROPEAN PATENT APPLICATION

(11) **EP 2 420 211 A1**
(43) Date of publication of application: **22.02.2012**
(21) Application number: 10173612.2
(22) Date of filing: 20.08.2010
(51) Int. Cl.: A61F 2/60, A61F 2/64, A61F 2/66, A61F 2/50

(54) **Joint for orthopedic articulations, such as ankle, knee and elbow braces or the like**

(71) Applicant: F.G.P. Srl, 37062 Dossobuono (VR) (IT)
(72) Inventor: Turrini, Alberto, 37062, Castel d'Azzano (VR) (IT); Ferrigolo, Moreno, 37062, Dossobuono (VR) (IT)
(74) Representative: Sandri, Sandro

(57) **Abstract**

A joint for orthopedic articulations that make up the frame of braces for the ankle, the knee, the elbow, the shoulder or the like, that is to say braces comprising means that allow the angular excursion of one sector (11) with respect to the other (12), said joint being equipped with means for controlling and limiting the relative angle of inclination between the two rods (11, 12), and consisting of pairs of mobile blocks (21), equipped with teeth, that operate inside tracks (18, 19), whose inner edges are fitted with teeth (20), present in the connecting rod (11), said mobile blocks (21) being kept in reciprocal contact with the fixed teeth (20) of the tracks (18, 19), and blocked therein in the required position by means of levers (22) of the cam type or the like and pivoted on the mobile blocks (21) by pins (23), said levers (22) being able to increase the distance between the teeth (20) of the tracks and the mobile blocks (21) allowing the release of the blocks and the subsequent adjustment of their position in the tracks.

## Description

### TECHNICAL FIELD

This invention concerns a joint for orthopedic articulations constructed by using a new system which allows graduated or linear flexion-extension control of an articulation.

The joint according to the invention can be fitted on orthopedic articulations which make up the frame of braces for ankles, knees, hips, elbows, shoulders or the like, that is to say braces comprising means that allow the angular and linear excursion of one sector with respect to the other, and comprise a ring nut type pivot associated with the two parts which are reciprocally mobile in an angular or linear direction. This ring nut can be adjusted by means of an innovative mechanical system which makes this solution advantageous from various points of view.

The joint according to the invention can be adapted to various situations and conditions, as well as being a new design and producible with relatively limited costs while ensuring maximum reliability and being ease to use.

This invention can be applied in the sector for the production of accessories for orthopedic products and braces mainly used in conservative, post-trauma, rehabilitation and post-operative therapy.

### BACKGROUND ART

It is known that the joint represents the pivot of the movement relative to every articulation. It is also known that subjects with post-operative, trauma or arthrosis problems of the articulations, for example the ankles, knees, hips, elbows or shoulders, need to be fitted with articulated braces or orthoses which comprise means for adjusting the angle of excursion of the affected articulations.

Braces that can be fitted to the articulations generally consist of a rigid frame designed to ensure adequate anchorage to the limb in order to avoid stress on the articulation and to limit or prevent torsion during walking.

For example, the frame of classic knee braces comprises means for constraining the brace to the femur and to the tibia in areas proximal to the knee and a structure connecting these means with a hinge positioned at the level of the knee.

To ensure that the limb has sufficient freedom of movement, the frame develops for a good distance laterally to the articulation, in order to allow reciprocal oscillation, in the case of the knee, between the femur and the tibia.

The means of constraint usually consist of straps, fitted with Velcro, or buckles suitable for adjustment of the locking tension and which, in the case of the knee for example, wrap around both the femur and the tibia of the user.

Some types of brace foresee the use of particular flexible hinges, inserted in the side arms of the brace's structure, and which make it possible to adjust the extent of the angular excursion of the articulation in question.

To limit the extent of the excursion, if necessary or in the event of particular problems, some hinges are equipped with screws or pins for adjustment of the angle of each hinge. The positioning of these pins or screws is not, however, a particularly easy task for the person wearing the brace to carry out independently, but requires considerable technical skill.

Such braces generally present a number of technical drawbacks. First of all, the angular strain exerted by the hinges fitted on the structure has a direct effect on the central joint.

This drawback becomes even more evident when the structure of the brace comprises an additional rigid side upright opposite the one equipped with hinges.

In general, the system for connecting the rods (fixed to the limb) and the central joint of known braces has a particularly delicate structure, being subject to most of the strain produced by the articulation.

In addition, its construction requires further technical effort for it to be industrialised, which makes it economically disadvantageous.

It is also difficult to precisely adjust the excursion of the working range of known braces, which has a negative effect on the general performance of these braces.

### DESCRIPTION OF THE INVENTION

This invention proposes to provide a joint for orthopedic articulations which comprises an innovative system of movement, limitation and control of the degrees of the relative movement. It can reduce or even eliminate the drawbacks described above

The invention also proposes to provide a joint equipped with an innovative system for controlling the relative angle of inclination (or of the linear travel) between two rods to which it is connected.

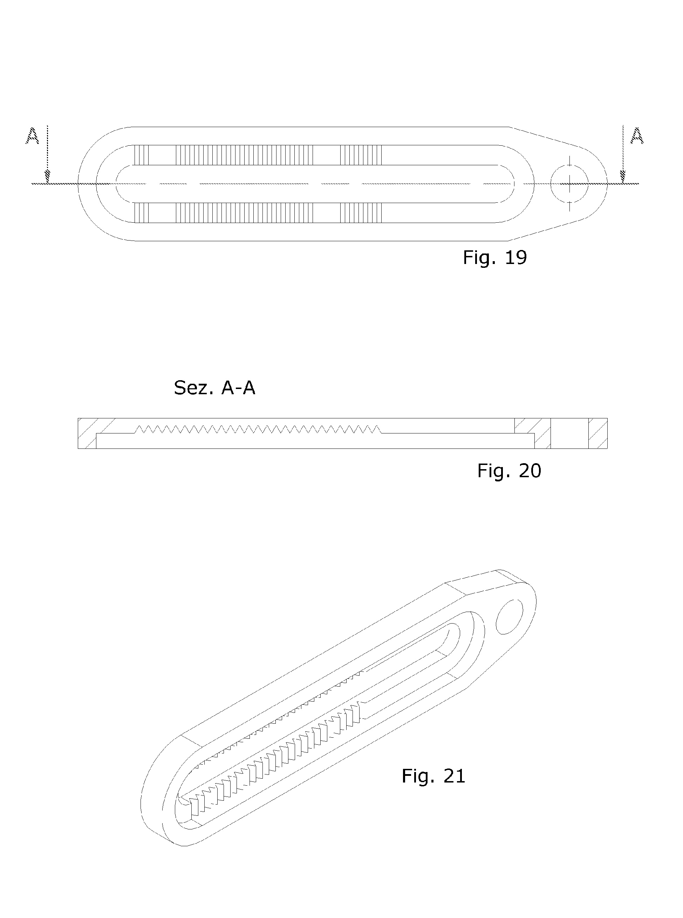

The invention can be fitted to two jointed rods connected by a pin (as in the example illustrated herein) or to two rods which slide linearly, one on top of the other, as can be seen in figures 19, 20 and 21. On examining the first case (the angular joint) it can be seen that this system is achieved by means of a joint for orthopedic articulations presenting the features described in the main claim.

The dependent claims describe advantageous embodiments of the invention.

The proposed aims are achieved, according to the invention, by a joint provided with means for activating and controlling its angular excursion, the main feature being the fact that the pivot consists of a ring nut and a counter ring nut or "plug" which is screwed and locked on to it.

This ring nut acts as a pivot and is inserted with moderate pressure in holes one of the two rods to block it. A connecting rod rotates freely around the axis of the ring nut, held axially in position by the plug.

In order to adjust the angle, the connecting rod is provided with two semicircular tracks equipped with radial teeth spaced a few degrees apart. Two blocks, one for each angular sector, also equipped with teeth, move along these tracks and engage with the teeth in the tracks.

### DESCRIPTION OF THE DRAWINGS

Other features and advantages of the invention will become clear on reading the description given below of one embodiment of the invention, provided as a non-binding example, with the help of the accompanying drawings in which:
- figure 1 is a schematic and prospective exploded view of the joint according to the invention;
- figure 2 is a schematic view of the rear part of the connecting rod;

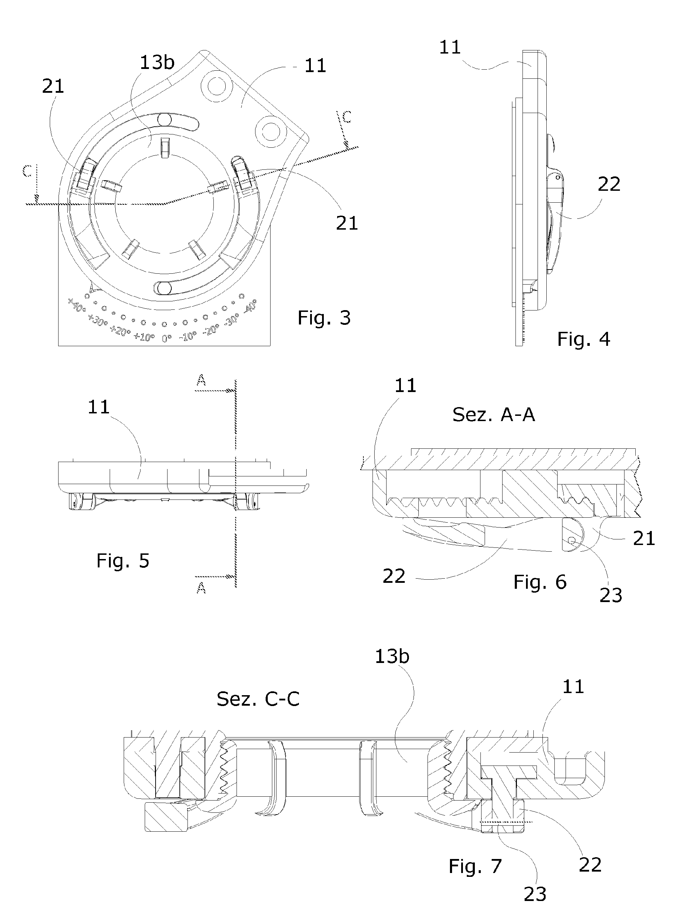

- figure 3 shows a schematic front view of the entire joint;
- figures 4 and 5 show schematic side views of the joint;
- figures 6 and 7 show views of the joint according to the indicated cross-sections;
- figures 8 to 11 show views of the joint in the various operating phases of the mobile blocks;
- figures 12 to 17 show views of the joint in various angular positions;
- figure 18 shows the joint according to the cross-section indicated in figure 17;
- figures 19 to 21 are schematic views of a possible solution of the invention with linear adjustment tracks, these tracks presenting parallel teeth (not radial ones as in the previous example) for engagement of the adjustment blocks.

### DESCRIPTION OF ONE EMBODIMENT OF THE INVENTION

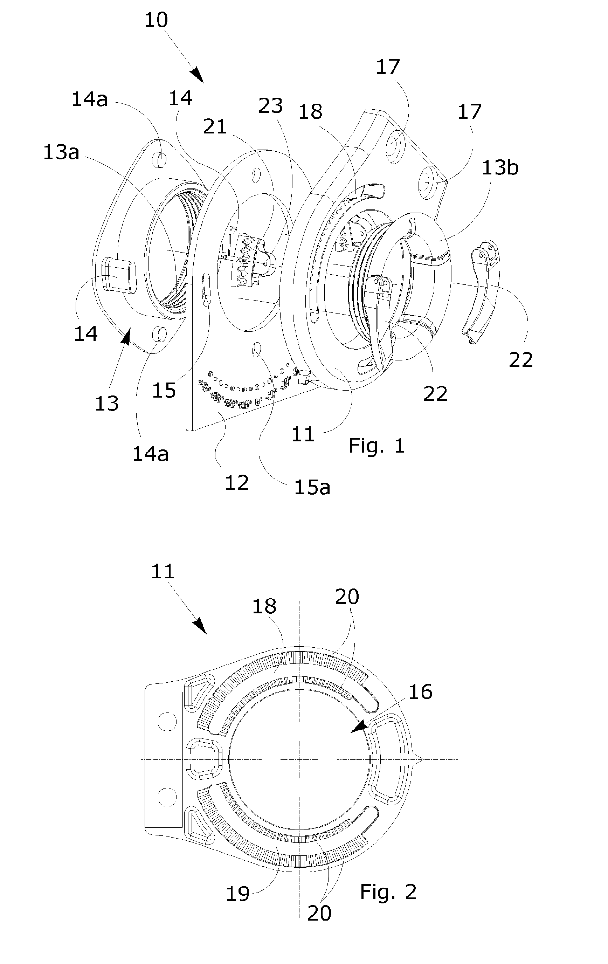

The joint according to the invention is indicated overall with the reference number 10 (figure 1) and is thus located between a connecting rod 11 attached to the first rod and the second rod 12 provided with appropriate holes. The ring nut 13 is coupled to the second rod by means of these holes. If the invention is applied to a foot brace, this second rod will act as the coupling for a shoe, while in the case of a knee brace, an elbow brace or the like, the shape of the second rod 12 will be similar to the first rod and positioned in an opposite direction.

The joint according to the invention presents a first feature whereby it is equipped with a large pivot consisting of the ring nut 13. This ring nut consists of an internally threaded part into which the plug 13b is screwed. The second feature is that it is provided with a control system for the relative angle of inclination between the two rods represented by the components 11 and 12 connected to it.

The plug 13b is screwed into and locked in the ring nut 13a which is constrained to the rod 12 by means of the holes 15 and 15a in which the pins 14 and 14a are inserted with moderate pressure on the component 12 to form a single body with it.

More specifically, the ring nut 13 comprises two bean-shaped fixed teeth 14 with wedge-shaped ends which are inserted in appropriate slots 15 in the component 12, in such a way that the two parts are fixed together and are held in place by the plug 13b.

The connecting rod 11 engages with the ring nut 13 and consists of a rounded body, or crown, which has a round central opening 16 in which the ring nut 13 is inserted. The connecting rod 11 can rotate freely around the axis of the ring nut, remaining held in position axially by the plug 13b. The connecting rod 11 also comprises a projection on which, by means of appropriate holes, the second rod of the brace is fixed.

To adjust the angular excursion of the connecting rod 11 with respect to the rod 12 (connected to the fixed ring nut in the case illustrated), the joint is equipped with an adjustment system as described below.

As can be seen in figure 2, the crown of the connecting rod 11 is provided with two semicircular tracks 18 and 19 whose inner edges are equipped with teeth 20 spaced, in the case illustrated here, 5° apart, and arranged radially with respect to the centre of rotation of the articulation.

The inner part of the crown of the connecting rod 11 houses two mobile blocks 21, one for each sector, provided with teeth which engage with the teeth of the connecting rod.

To maintain the reciprocal contact between the teeth of the mobile blocks 21 and the fixed teeth 20, the mobile blocks 21 are equipped with two cam levers 22 pivoted on the mobile blocks 21 by means of pins 23.

When in the rest position, that is to say in the position of contact between the mobile blocks 21 and the toothed tracks 20 of the connecting rod, these cam levers are adjacent to the outer surface of the connecting rod 11.

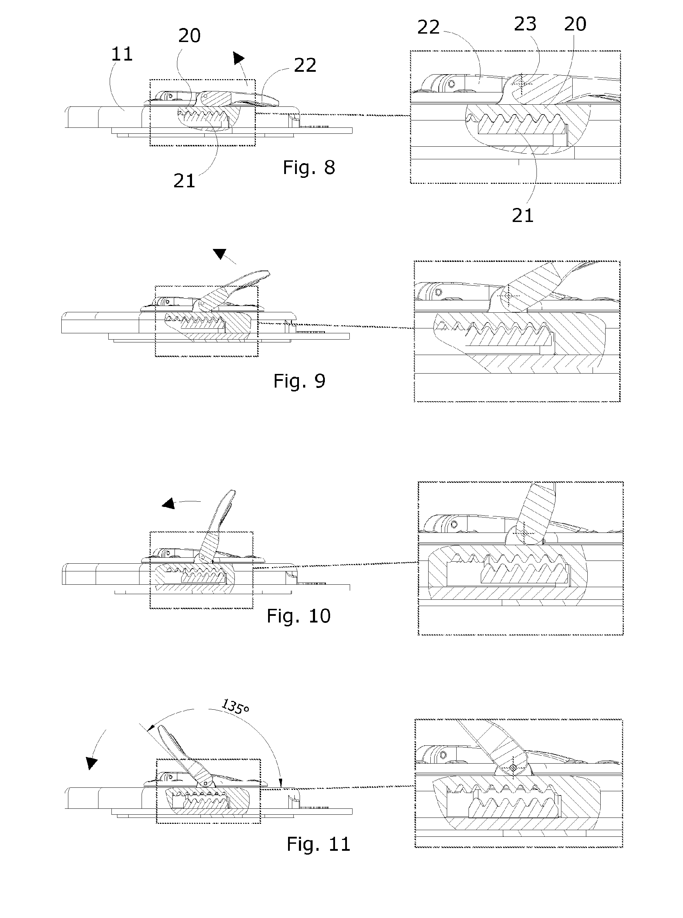

By operating these levers, it is possible to increase the distance between the toothed tracks 20 and the mobile blocks 21, allowing their release and the subsequent adjustment of the angular position, as can be seen in figures 8 to 11.

As can be seen in figure 11, when the lever is completely open (135°) there is the maximum distance between the mobile blocks and the toothed tracks of the connecting rods. In this position the mobile blocks 21 will just skim the surface below.

To allow adjustment of the angles of flexion (between 0° and -40°) and extension (between 0° and +40°), the mobile blocks strike against two fixed teeth 14 on the ring nut 13, which, passing through the slots 15 in the component 12, penetrate the two semicircular tracks 18 and 19.

The two mobile blocks 21 therefore strike against these teeth, blocking the angular rotation of the connecting rod 11, being firmly engaged with it by means of the teeth 20, and limiting the angular movements of the joint.

By positioning the mobile blocks 21 so that they simultaneously engage with both teeth 14 of the ring nut, the joint can be immobilised and blocked in any position between -40° and +40° (in steps of 5° in this example).

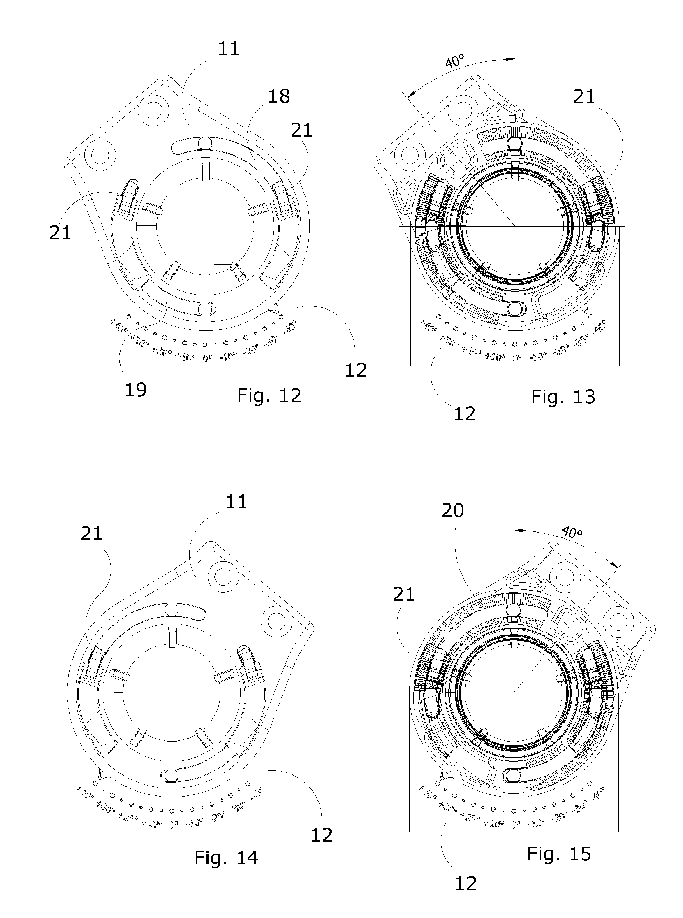

Figures 12 and 13 show the joint blocked in the -40° position while figures 14 and 15 show it blocked in the +40° position.

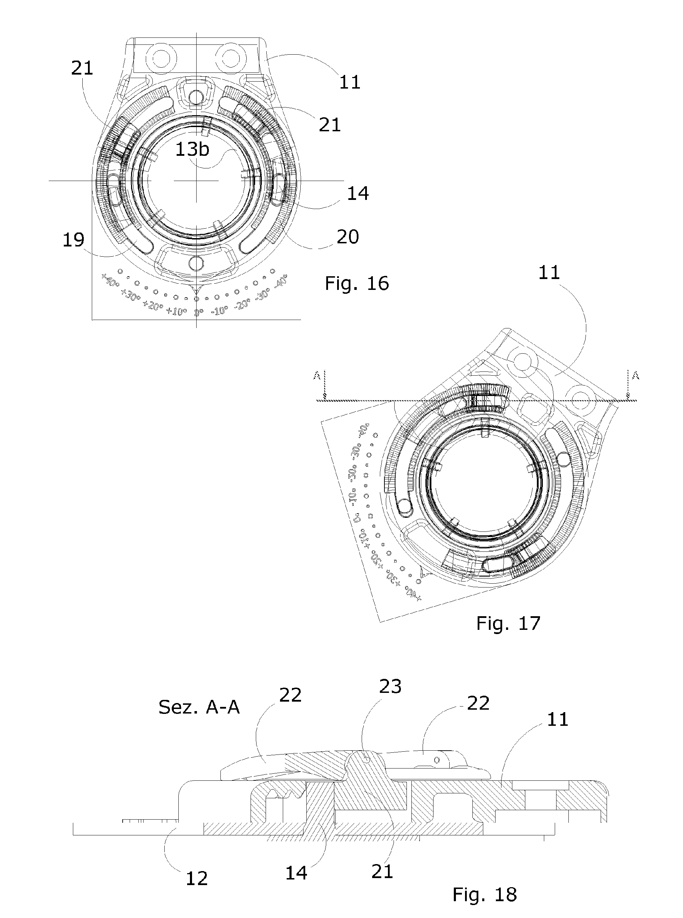

In figure 16, the joint, in the 0° position, is adjusted so that it is free to move between -10° and +30°.

As can be seen in figure 18, to ensure that the levers 22 remain adherent to the connecting rod 11, the levers are equipped with a projection while the relative block has a notch which engages with the projection.

As mentioned above, the joint 10 according to the invention is positioned between the two rods which, in the case illustrated here, are firmly fixed to the connecting rod 11 (the tibial rod) and to the ring nut 13 fixed to the shoe of a foot brace. In the case of a knee brace or an elbow brace or the like, the second rod 12 is shaped externally in the same way as the first rod and positioned in the opposite direction.

According to further embodiments of the invention, it is foreseen that, through the use of appropriate auxiliary connecting rods, the semicircular tracks 18, 19 can be linear in shape (see figures 19 to 21), and the adjustment movement of the blocks 21 is rectilinear.

The invention is described above with reference to a preferred embodiment. It is nevertheless clear that the invention is susceptible to numerous variations which lie within the scope of its disclosure, within the framework of technical equivalents.

## Claims

1. A joint for orthopedic articulations that make up the frame of braces for the ankle, the knee, the elbow, the shoulder or the like, that is to say braces comprising means that allow the angular excursion of one sector (11) with respect to the other (12), said joint being **characterised in that** it is equipped with means for controlling and limiting the relative angle of inclination between the two rods (11, 12), and consisting of pairs of mobile blocks (21), equipped with teeth, that operate inside tracks (18, 19), whose inner edges are fitted with teeth (20), present in the connecting rod (11), said mobile blocks (21) being kept in reciprocal contact with the fixed teeth (20) of the tracks (18, 19), and blocked therein in the required position by means of levers (22) of the cam type or the like and pivoted on the mobile blocks (21) by pins (23), said levers (22) being able to increase the distance between the teeth (20) of the tracks and the mobile blocks (21) allowing the release of the blocks and the subsequent adjustment of their position in the tracks.

2. A joint for orthopedic articulations according to the foregoing claim, **characterised in that** its pivot, associated with the two reciprocally mobile parts in an angular direction, is the ring nut type (13) consisting of a ring nut component (13a) and a plug (13b) designed to accommodate, with the possibility of angular movement, the crown of the connecting rod (11), said ring nut (13a) comprising wedge-shaped fixed teeth (14) which are inserted in appropriate slots (15) of the element (12), in such a way that the two parts are joined together.

3. A joint for orthopedic articulations according to either of the foregoing claims, **characterised in that** the connecting rod (11) consists of a rounded body, or crown, with a central round opening (16), in which the ring nut (13) is inserted, to rotate freely around the axis of the ring nut, remaining held in position axially by the plug (13b), said connecting rod (11) comprising a sector protruding at right angles to the rotation axis, equipped with holes in which the brace rod is inserted.

4. A joint for orthopedic articulations according to any of the foregoing claims, **characterised in that** the crown of the connecting rod (11) is provided with two semicircular tracks (18, 19) whose inner edges are equipped with teeth (20) which are positioned radially with respect to the centre of rotation of the articulation.

5. A joint for orthopedic articulations according to any of the foregoing claims, **characterised in that** the crown of the connecting rod (11) houses two mobile blocks (21), one for each sector, with teeth that engage with the teeth (20) of the connecting rod (11).

6. A joint for orthopedic articulations according to any of the foregoing claims, **characterised in that** by operating the levers (22) it is possible to increase the distance between the track teeth (20) and the mobile blocks (21) allowing their release and the subsequent adjustment of the angular position.

7. A joint for orthopedic articulations according to any of the foregoing claims, **characterised in that** the mobile blocks strike against the two fixed teeth (14) on the ring nut (13), which, passing through the slots (15) in the element 12, penetrate the two semicircular tracks (18, 19).

8. A joint for orthopedic articulations according to any of the foregoing claims, **characterised in that**, when in the closed position, the levers (22) remain adherent to the connecting rod (11) by means of a projection on the lever and a relative notch in the block which engage with each other.

9. A joint for orthopedic articulations according to any of the foregoing claims, **characterised in that** it is positioned between the first (11) and second (12) rods which, in the case illustrated here, consist respectively of the connecting rod (11) on which the tibial rod is fitted and of the ring nut (13) on which the shoe of a foot brace is fitted (represented by the element (12)), while in the case of a knee brace or an elbow brace or the like, the second support rod (12) of the brace is shaped externally in the same way as the first rod and positioned in the opposite direction.

10. A joint for orthopedic articulations according to any of the foregoing claims, **characterised in that**, through the use of appropriate auxiliary connecting rods, the semicircular tracks (18, 19) can be linear in shape and the adjustment movement of the blocks (21) is rectilinear.
